# EUROPÄISCHE PATENTANMELDUNG

(11) **EP 3 091 357 A1**
(43) Veröffentlichungstag der Anmeldung: **09.11.2016**
(21) Anmeldenummer: 16166164.0
(22) Anmeldetag: 20.04.2016
(51) Int. Cl.: G01N 33/497, A61B 5/08, G01N 1/22, G01N 27/02

(54) **FILTER FÜR EIN ATEMLUFTANALYSEGERÄT, ATEMLUFTANALYSEGERÄT UND VERFAHREN ZUM ÜBERWACHEN EINES FILTERS**

(30) Priorität: 06.05.2015 DE 102015208443
(71) Anmelder: ROBERT BOSCH GMBH, 70442 Stuttgart (DE)
(72) Erfinder: Vogel, Heike, 71394 Kernen Im Remstal (DE); Herlt, Christina, 70736 Fellbach (DE); Sahner, Kathy, 68167 Mannheim (DE); Wittmann, Hubert, 92242 Hirschau (DE)

(57) **Zusammenfassung**

Die Erfindung betrifft einen Filter (100) für ein Atemluftanalysegerät (200), wobei der Filter (100) ein Filtergehäuse (104), in dem ein Konvertermaterial (108) zwischen einer Gaseintrittsöffnung (102) und einer Gasaustrittsöffnung (106) angeordnet oder anordenbar ist und einen Sensor (110) aufweist, der in dem Filtergehäuse (104) angeordnet ist und zum Erfassen einer Charakteristik zumindest eines Teils des Konvertermaterials (108) eine erste Elektrode (112) und eine zweite Elektrode (114) aufweist, zwischen denen der zumindest eine Teil des Konvertermaterials (108) angeordnet oder anordenbar ist.

## Beschreibung

### Stand der Technik

Die Erfindung geht aus von einem Filter für ein Atemluftanalysegerät, einem Atemluftanalysegerät oder einem Verfahren nach Gattung der unabhängigen Ansprüche.

Die EP 1384069 B1 behandelt das Gebiet der Atemgasanalyse. Dabei bläst ein Patient in ein Gerät, um die Konzentration von verschiedenen Gasen in der Atemluft zu bestimmen.

### Offenbarung der Erfindung

Vor diesem Hintergrund werden mit dem hier vorgestellten Ansatz ein Filter für ein Atemluftanalysegerät, ein Atemluftanalysegerät sowie ein Verfahren zum Überwachen eines Filters gemäß den Hauptansprüchen vorgestellt. Durch die in den abhängigen Ansprüchen aufgeführten Maßnahmen sind vorteilhafte Weiterbildungen und Verbesserungen der im unabhängigen Anspruch angegebenen Vorrichtung möglich.

Wenn eine Charakteristik zumindest eines Teils eines in einem Filter verwendeten Konvertermaterials direkt in dem Filter erfasst werden kann, so kann aus der erfassten Charakteristik auf einen Funktionszustand des Konvertermaterials und somit des Filters geschlossen werden.

Es wird ein Filter für ein Atemluftanalysegerät vorgestellt, wobei der Filter die folgenden Merkmale aufweist:
ein Filtergehäuse, in dem ein Konvertermaterial zwischen einer Gaseintrittsöffnung und einer Gasaustrittsöffnung angeordnet ist; und
einen Sensor, der in dem Filtergehäuse angeordnet ist und zum Erfassen einer Charakteristik zumindest eines Teils des Konvertermaterials eine erste Elektrode und eine zweite Elektrode aufweist, zwischen denen zumindest ein Teil des Konvertermaterials (108) angeordnet oder anordenbar ist.

Der Filter ist zum Einsatz für ein Atemluftanalysegerät aber beispielsweise auch für eine Klimaanlage geeignet. Unter einem Atemluftanalysegerät kann ein medizintechnisches Gerät zum Analysieren von Bestandteilen der Atemluft eines Patienten verstanden werden. Die Atemluft kann als Atemgas bezeichnet werden. Ein Filter kann eine Einrichtung zum Entfernen eines bestimmten Anteils eines Mediums sein. Hier ist der Filter dazu ausgebildet, zumindest einen Bestandteil der Atemluft durch eine Konversion in zumindest einen veränderten Bestandteil zu ändern. Ein Filtergehäuse kann eine Hülle des Filters sein. Das Filtergehäuse gibt dem Filter seine strukturelle Integrität. Die Gaseintrittsöffnung und die Gasaustrittsöffnung können als Schnittstellen zu dem Atemluftanalysegerät bezeichnet werden. Der Filter kann eine vorgegebene Durchströmungsrichtung aufweisen. Die Konversion erfolgt unter Verwendung des Konvertermaterials. Bei der Konversion reagiert der Bestandteil der Atemluft mit dem Konvertermaterial. Dabei wird der Bestandteil umgewandelt und das Konvertermaterial wird verbraucht. Unter einer Charakteristik kann ein Merkmal, beispielsweise eine elektrische Eigenschaft des Konvertermaterials verstanden werden. Die Charakteristik kann von einem Funktionszustand des Konvertermaterials, beispielsweise verbraucht oder unverbraucht, abhängig sein. Somit kann sich die Charakteristik eines verbrauchten Konvertermaterials von der Charakteristik eins unverbrauchten Konvertermaterials unterscheiden. Die Charakteristik kann unter Verwendung der Elektroden bestimmt werden und in einem elektrischen Signal abgebildet werden. Somit kann der Sensor ausgebildet sein, um ein die Charakteristik und somit einen Funktionszustand des Konvertermaterials repräsentierendes Signal bereitzustellen. Im befüllten Zustand des Filters können die Elektroden des Filters durch den zumindest einen Teil des Konvertermaterials voneinander beabstandet sein.

Der hier vorgestellte Ansatz eignet sich zur Anwendung auf solche Fälle, in denen ein Analyt vor der Messung im Atemluftanalysegerät chemisch verändert wird, beispielsweise durch Reduktion oder Oxidation durch sich verbrauchende Chemikalien, wie beispielsweise Kaliumpermanganat für die Oxidation, im folgenden Konverter genannt. Das Konvertermaterial kann aus dem Konverter bestehen oder einen solchen Konverter umfassen. In Medizintechnik-Produkten kann diese Methode einem Katalysator vorgezogen werden, da Katalysatoren zumeist hohe Temperaturen benötigten, die in den entsprechenden Systemen schwer umgesetzt werden können.

Konverter für medizintechnische Anwendungen oder für die Luftfilterung in Klimaanlagen können auf Pulver oder kleinen Kugeln aus Silicagel oder Aluminiumoxid aufgebracht werden, um die Oberfläche zu vergrößern.

Der Konverter verbraucht sich, was bei wiederholter Nutzung und/oder zu langer Standzeit zu einer unvollständigen Konversion des Analyten und somit zu einer Verfälschung des Messergebnisses führen kann. Um dieser unvollständigen Konversion vorzubeugen, kann der Konverter regelmäßig ausgetauscht werden. Bisher erfolgt das beispielsweise nach x Messungen und/oder einem Zeitraum y. Eine direkte Überwachung, ob die Funktionsfähigkeit des Konverters noch gegeben ist bzw. eine Rückmeldung, sobald diese gefährdet ist, wäre vorteilhaft.

Durch die hier vorgestellte Messeinrichtung am Filter kann eine Rückmeldung über die Funktionstüchtigkeit des Konverters gegeben werden. Dadurch können Fehlmessungen durch unvollständige Konversion in verbrauchtem Konverter vermieden werden. Ein Wechsel des Konverters ist nur im Bedarfsfall erforderlich, was einen finanziellen und zeitlichen Vorteil bietet, da die Konverterkartusche im Gerät verbaut ist und von einem Servicetechniker gewechselt wird.

Durch die Rückmeldung über die tatsächliche Funktionsfähigkeit des Konverters kann eine Restunsicherheit ausgeschlossen werden.

Der Sensor kann mit dem zumindest einen Teil des Konvertermaterials beschichtet sein. Damit verhält sich das Konvertermaterial auf dem Sensor näherungsweise wie benachbartes Konvertermaterial auf einem, den Sensor umgebenden, Trägermaterial. Die Charakteristiken der benachbarten Konvertermaterialien stimmen somit im Wesentlichen überein. Auf diese Weise ist es ausreichend, die Charakteristik des sich im Erfassungsbereich des Sensors befindlichen Konvertermaterials zu erfassen, um auf den Funktionszustand des Filters schließen zu können.

Der Sensor kann mit einem Abstand zu der Gasaustrittsöffnung in dem Filtergehäuse angeordnet sein. Der Abstand kann zumindest einer kritischen Schütthöhe des Konvertermaterials vor der Gasaustrittsöffnung entsprechen. Damit kann der Sensor erfassen, ob der Verbrauch des Konvertermaterials bis zu der kritischen Schütthöhe vorgedrungen ist. Die kritische Schütthöhe repräsentiert eine Entfernung von der Gasaustrittsöffnung in Richtung der Gaseintrittsöffnung. Wenn das Konvertermaterial bis auf die kritische Schütthöhe verbraucht ist, sollte der Filter vorteilhafterweise zeitnah ausgewechselt werden. In der kritischen Schütthöhe kann eine Sicherheitsreserve an Konvertermaterial vorgesehen sein.

Die erste Elektrode kann im Bereich der Gaseintrittsöffnung angeordnet sein. Die zweite Elektrode kann im Bereich der Gasaustrittsöffnung angeordnet sein. Damit kann die Charakteristik des gesamten Konvertermaterials erfasst werden.

Die Elektroden können als Gitterelektroden oder alternativ als Fingerelektroden ausgeführt sein. Gitterelektroden können beispielsweise an der Gaseintrittsöffnung und der Gasaustrittsöffnung angeordnet sein. Die Fingerelektroden können als interdigitale Fingerelektroden ausgeführt sein und wechselseitig miteinander kämmen. So können die Fingerelektroden einen definierten Abstand zueinander aufweisen, in dem die Charakteristik sicher bestimmt werden kann.

Die Elektroden können in einer Wand des Filtergehäuses ausgeformt sein. Auf diese Weise können der Sensor und gegebenenfalls elektrische Leitungen zum Kontaktieren des Sensors in dem Filtergehäuse integriert sein. Dadurch ist kein separates Sensorelement erforderlich, sodass die Herstellung des Filters vereinfacht wird. Zudem ist eine Position des Sensors innerhalb des Filtergehäuses vordefiniert und damit bekannt.

Der Sensor kann als ein Impedanzsensor ausgeführt sein. Eine Impedanz des Konvertermaterials kann durch eine Wechselspannung zwischen den Elektroden gemessen werden. Somit kann die Charakteristik eine Impedanz des Konvertermaterials repräsentieren. Alternativ kann der Sensor beispielsweise als ein kapazitiver Sensor ausgeführt sein.

Der Filter kann eine Impedanzerfassungseinrichtung aufweisen, die dazu ausgebildet ist, die Charakteristik als eine Impedanz des zumindest einen Teil des Konvertermaterials unter Verwendung eines Signals des Sensors zu erfassen und beispielsweise in einem Impedanzwert abzubilden. Die Impedanzerfassungseinrichtung kann eine elektrische Versorgungsspannung an den Sensor bereitstellen. Insbesondere kann die Impedanzerfassungseinrichtung ausgebildet sein, um zum Erfassen der Impedanz eine Wechselspannung mit einer variablen Frequenz an die Elektroden des Sensors bereitzustellen.

Der Filter kann eine Bereitstellungseinrichtung aufweisen, die ausgebildet ist, um unter Verwendung der Charakteristik des Konvertermaterials ein Zustandssignal bereitzustellen, das einen Funktionszustand des Filters repräsentiert. Beispielsweise kann die Bereitstellungseinrichtung ausgebildet sein, um ein von dem Sensor oder unter Verwendung des Sensors bereitgestelltes Signal, das die Charakteristik des Konvertermaterials abbildet, zu erfassen und unter Verwendung des Signals das Zustandssignal bereitzustellen. Beispielsweise kann die Bereitstellungseinrichtung als eine Vergleichseinrichtung ausgeführt sein, die dazu ausgebildet ist, die unter Verwendung des Sensors erfasste Charakteristik mit einer Sollcharakteristik oder einem Schwellenwert zu vergleichen. Die Vergleichseinrichtung kann ausgebildet sein, um abhängig von einem Vergleichsergebnis des Vergleichs das Zustandssignal bereitzustellen, das je nach Ausführungsform einen unverbrauchten oder verbrauchten Zustand des Konvertermaterials anzeigen kann. Beispielsweise kann die Vergleichseinrichtung ausgebildet sein, um das Zustandssignal bereitzustellen, wenn die Charakteristik außerhalb eines vordefinierten Toleranzbereichs liegt.

Weiterhin wird ein Atemluftanalysegerät mit einem Filter gemäß dem hier vorgestellten Ansatz vorgestellt. Der Filter kann als eine austauschbare Kartusche in dem Atemluftanalysegerät eingesetzt sein.

Ferner wird ein Verfahren zum Überwachen eines Filters für ein Atemluftanalysegerät vorgestellt, wobei der Filter ein Filtergehäuse aufweist, in dem ein Konvertermaterial zwischen einer Gaseintrittsöffnung und einer Gasaustrittsöffnung angeordnet ist, und wobei das Verfahren den folgenden Schritt aufweist:
Erfassen einer Charakteristik eines Konvertermaterials des Filters unter Verwendung eines Sensors, der in dem Filtergehäuse angeordnet ist und eine erste Elektrode und eine zweite Elektrode aufweist, zwischen denen der zumindest ein Teil des Konvertermaterials angeordnet ist.

Das Verfahren kann einen Schritt des Bereitstellens umfassen, in dem eine Zustandsinformation des Filters und alternativ oder ergänzend eine Wartungsinformation für den Filter unter Verwendung der Charakteristik bereitgestellt wird. Durch eine solche bereitgestellte Information, beispielsweise in Form eines Zustandssignals, kann der Filter überwacht werden. Die Überwachung kann genutzt werden, um den Filter zu wechseln, wenn das Konvertermaterial einen vorbestimmten Zustand erreicht hat.

Ausführungsbeispiele der Erfindung sind in den Zeichnungen dargestellt und in der nachfolgenden Beschreibung näher erläutert. Es zeigt:
Fig. 1 eine schematische Darstellung eines Filters für ein Atemluftanalysegerät gemäß einem Ausführungsbeispiel;
Fig. 2 ein Blockschaltbild eines Atemluftanalysegeräts mit einem Filter gemäß einem Ausführungsbeispiel;
Fig. 3 eine Darstellung eines Filters gemäß einem Ausführungsbeispiel;
Fig. 4 eine Darstellung eines Sensors gemäß einem Ausführungsbeispiel;
Fig. 5 eine weitere Darstellung eines Sensors gemäß einem Ausführungsbeispiel;
Fig. 6 Impedanzkurven von Impedanzsensoren gemäß einem Ausführungsbeispiel;
Fig. 7 Alterungskurven eines Konvertermaterials gemäß einem Ausführungsbeispiel; und
Fig. 8 ein Ablaufdiagramm eines Verfahrens zum Überwachen eines Filters für ein Atemluftanalysegerät gemäß einem Ausführungsbeispiel.

In der nachfolgenden Beschreibung günstiger Ausführungsbeispiele der vorliegenden Erfindung werden für die in den verschiedenen Figuren dargestellten und ähnlich wirkenden Elemente gleiche oder ähnliche Bezugszeichen verwendet, wobei auf eine wiederholte Beschreibung dieser Elemente verzichtet wird.

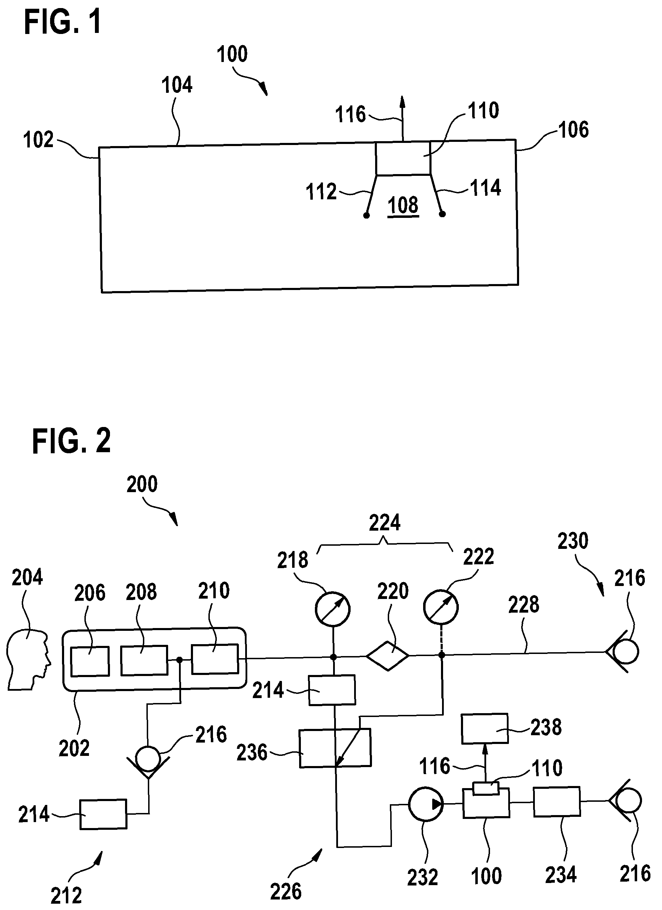

Fig. 1 zeigt eine schematische Darstellung eines Filters 100, der beispielhaft für ein Atemluftanalysegerät eingesetzt werden kann, gemäß einem Ausführungsbeispiel. Der Filter 100 ist dazu ausgebildet, einen Luftstrom in dem Atemluftanalysegerät zu filtern. Der Filter 100 ist eine austauschbare Baugruppe des Atemluftanalysegeräts und kann als Filterkartusche 100 bezeichnet werden.

Der Luftstrom tritt im Betrieb des Atemluftanalysegeräts als ungefilterter Luftstrom durch eine Gaseintrittsöffnung 102 in ein Filtergehäuse 104 des Filters 100 ein und verlässt nach dem Filtern das Filtergehäuse 104 als gefilterter Luftstrom durch eine Gasaustrittsöffnung 106. In dem Filtergehäuse 104 ist ein Konvertermaterial 108 angeordnet. Das Konvertermaterial 108 ist dazu ausgebildet, den Luftstrom zu filtern. Das Konvertermaterial 108 kann beispielsweise auf einem Trägermaterial aufgebracht sein. Das Trägermaterial stellt eine möglichst große Oberfläche für das Konvertermaterial 108 zur Verfügung. Dazu kann das Trägermaterial, mit dem Konvertermaterial 108 darauf, als Schüttung in das Filtergehäuse 104 eingebracht sein. Die Schüttung füllt dabei zumindest einen Großteil des Filtergehäuses 104 zwischen der Gaseintrittsöffnung 102 und der Gasaustrittsöffnung 106 vollständig aus.

Das Konvertermaterial 108 reagiert mit zumindest einer chemischen Spezies, indem sich ein Bestandteil des Konvertermaterials 108 mit der chemischen Spezies verbindet und/oder indem sich ein Bestandteil der Spezies mit dem Konvertermaterial 108 verbindet. Dadurch verändern sich das Konvertermaterial 108 und die chemische Spezies. Das Konvertermaterial 108 wird verbraucht. Die chemische Spezies wird in eine gewollte Spezies konvertiert oder adsorbiert. Eine Adsorption erfolgt beispielsweise für den Fall der Verwendung von Aktivkohle.

Wenn die chemische Spezies in dem Luftstrom vorhanden ist, reagiert sie mit dem ersten unverbrauchten Konvertermaterial 108, mit dem die Spezies in Berührung kommt. Dabei wird das unverbrauchte Konvertermaterial 108, das am nächsten an der Gaseintrittsöffnung 102 angeordnet ist, zuerst verbraucht. Wenn der Filter 100 neu ist, ist im Wesentlichen alles Konvertermaterial 108 unverbraucht. Im Laufe der Benutzung wird das Konvertermaterial 108 von der Gaseintrittsöffnung 102 zu der Gasaustrittsöffnung verbraucht. Wenn nur noch wenig unverbrauchtes Konvertermaterial 108 im Bereich der Gasaustrittsöffnung 106 im Filter 100 vorhanden ist, sinkt die Wahrscheinlichkeit, dass alle in dem Luftstrom vorhandene Spezies in Kontakt mit unverbrauchtem Konvertermaterial 108 kommt. Damit steigt die Wahrscheinlichkeit, dass ein Teil der Spezies ohne konvertiert zu werden durch den Filter 100 gelangt. Wenn der Luftstrom durch eine definierte Mindeststrecke unverbrauchtes Konvertermaterial 108 strömt, wird die chemische Spezies mit einer bestimmungsgemäßen Mindestwahrscheinlichkeit in die gewollte Spezies konvertiert.

Um eine bestimmungsgemäße Funktionsfähigkeit des Filters 100 zu erfassen, ist in dem Filtergehäuse 104 ein Sensor 110 zum Erfassen einer Charakteristik des Konvertermaterials 108 angeordnet. Die Charakteristik repräsentiert hier, ob das Konvertermaterial 108 unverbraucht oder verbraucht ist.

Der Sensor 110 weist eine erste Elektrode 112 und eine zweite Elektrode 114 auf. Zwischen den Elektroden 112, 114 ist ein Zwischenraum. In dem Zwischenraum ist Konvertermaterial 108 angeordnet. Durch die Elektroden 112, 114 wird die Charakteristik des Konvertermaterials 108 zwischen den Elektroden 112, 114 erfasst. Der Sensor 110 ist dazu ausgebildet, die Charakteristik in einem elektrischen Signal 116 abzubilden.

Der Sensor 110 kann neben den Elektroden 112, 114 eine Messschaltung umfassen, die beispielsweise ausgebildet ist, um angeregt durch eine an den Sensor 110 angelegte Betriebsspannung eine Impedanzmessung durchzuführen. Alternativ kann eine solche Messschaltung, beispielsweise entsprechend der in Fig. 2 gezeigten Steuerelektronik, separat zu dem Filter 100 angeordnet sein und über eine Schnittstelle des Filtergehäuses 104 mit dem Sensor 110 gekoppelt sein.

Der Sensor 110 kann als ein diskretes Bauteil ausgeführt sein, das in das Filtergehäuse 104 eingeführt worden sein kann. Alternativ kann der Sensor 110 als Teil des Filtergehäuses 104 ausgeführt sein, beispielsweise in Form eines Aufdrucks von Strukturelementen des Sensors 110 auf eine dem Konvertermaterial 108 zugewandte Innenseite des Filtergehäuses 104.

Der Teil des Konvertermaterials 108, dessen Charakteristik von dem Sensor 110 erfasst werden kann, kann während der Befüllung des Filtergehäuses 104 mit dem Konvertermaterial 108 zwischen den Elektroden 112, 114 angeordnet werden. Alternativ kann dieser Teil des Konvertermaterials 108 als zusätzliches Konvertermaterial 108, in Form eines Bestandteil des Sensors 110, zusammen mit dem Sensor 110 in das Filtergehäuse 104 integriert oder eingeführt werden, also vor oder nach der Befüllung des Filtergehäuses 104 mit dem eigentlichen Konvertermaterial 108 in das Filtergehäuse 104 eingebracht werden.

Fig. 2 zeigt ein Blockschaltbild eines Atemluftanalysegeräts 200 mit einem Filter 100 gemäß einem Ausführungsbeispiel. Der Filter 100 entspricht dabei im Wesentlichen dem Filter in Fig. 1. Das Atemluftanalysegerät 200 als Gesamtsystem weist ein austauschbares Mundstück 202 auf, durch das ein Patient 204 einen Luftstrom in das Atemluftanalysegerät 200 einblasen kann. Das Mundstück 202 weist eine Einblasöffnung 206, einen Bakterienfilter 208 und einen Lufttrockner 210 auf. Der Luftstrom strömt dabei vom Patienten 204 durch die Einblasöffnung 206, durch den Bakterienfilter 208 und durch die Lufttrocknungseinrichtung 210. In der Lufttrocknungseinrichtung 110 ist ein Trocknungsmittel angeordnet. Zwischen dem Bakterienfilter 208 und der Lufttrocknungseinrichtung 210 ist ein Umgebungslufteinlass 212 angeschlossen. Der Umgebungslufteinlass 212 weist einen Nullluftfilter 214 und ein Rückschlagventil 216 auf. Das Rückschlagventil 216 verhindert, dass Atemluft aus dem Mundstück 202 durch den Umgebungslufteinlass 212 ausströmt.

Nach dem Mundstück 202 ist ein Drucksensor 218 angeordnet. Der Drucksensor 218 ist zwischen dem Mundstück 202 und einer Drossel 220 angeordnet. Die Drossel 220 erzeugt einen Ausatemwiderstand für den Patienten 204.

Nach der Drossel 220 kann ein optionaler Drucksensor 222 angeordnet sein. Durch den Drucksensor 218 und den optionalen Drucksensor 222 kann ein Differenzialdruck 224 an der Drossel 220 erfasst werden.

Nach der Drossel 220 zweigt ein Sensorpfad 226 von einem Hauptpfad 228 des Atemluftanalysegeräts 200 ab. Der Hauptpfad 228 verläuft nach der Abzweigung des Sensorpfads 226 direkt zu einem Umgebungsluftauslass 230, an dem ein weiteres Rückschlagventil 216 angeordnet ist. Das weitere Rückschlagventil 216 verhindert ein Einströmen von Umgebungsluft in den Hauptpfad 228.

Im Sensorpfad 226 ist eine Pumpe 232 angeordnet, die einen definierten Luftstrom aus dem Hauptpfad 228 absaugt und durch den Filter 100 beziehungsweise Konverter 100 zu einem Sensor 234 leitet. Nach dem Sensor 234 wird der abgezweigte Luftstrom ebenfalls über ein Rückschlagventil 216 durch einen weiteren Umgebungsluftauslass 230 in die Umgebung abgelassen.

Vor der Pumpe 232 ist ein Ventil 236 angeordnet, mit dem ein Bypass zum Hauptpfad 228 vor der Drossel 220 geschaltet werden kann. In dem Bypass ist ein weiterer Nullluftfilter 214 angeordnet.

Eine Steuerelektronik 238 des Atemluftanalysegeräts 200 ist mit dem Sensor 110 des Filters 100 verbunden und empfängt das Signal 116. Die Steuerelektronik ist dazu ausgebildet, das Signal 116 auszuwerten und eine Diagnoserückmeldung am Gerät 200 für einen Bediener des Atemluftanalysegeräts 200 bereitzustellen. Beispielsweise ist die Steuerelektronik 238 als eine Bereitstellungseinrichtung zum Bereitstellen eines den Zustand des Filters 100 anzeigenden Zustandssignals ausgeführt. Dazu kann die Steuerelektronik 238 ausgebildet sein, um das Signal 116 mit einem Schwellenwert zu vergleichen, um den Zustand des Konvertermaterials des Filters 100 beurteilen zu können. Alternativ kann die Steuerelektronik 238 ausgebildet sein, um das Signal 116 als Zustandssignal auszugeben.

Somit kann das elektrische Sensorsignal 116 zur digitalen Rückmeldung, ob der Konverter des Filters 100 in Ordnung oder nicht in Ordnung ist, an den Nutzer über den Zustand des Konverters des Filters 100 verwendet werden, beispielsweise über eine Serviceanzeige oder eine Warnlampe.

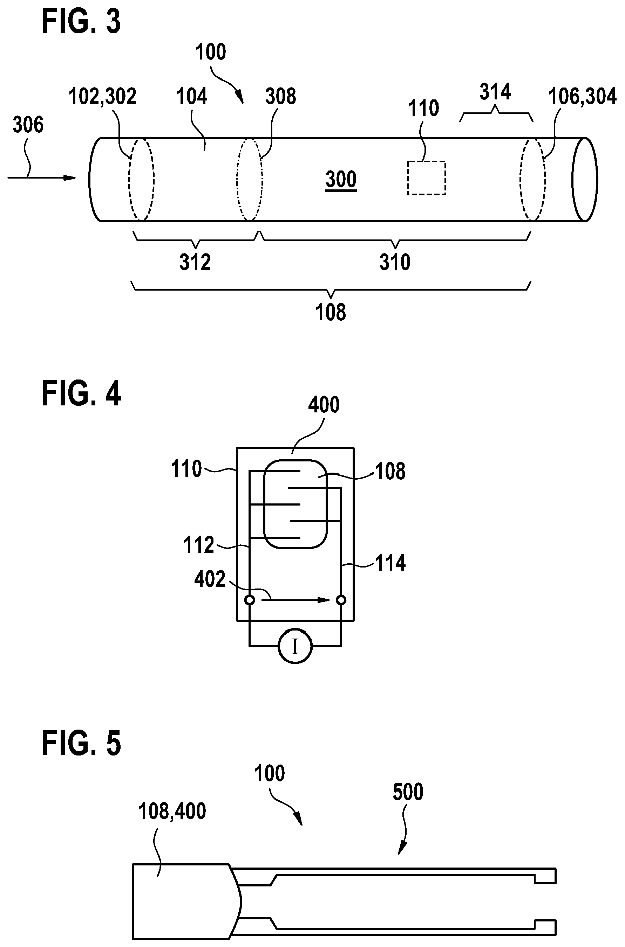

Fig. 3 zeigt eine Darstellung eines Filters 100 gemäß einem Ausführungsbeispiel. Der Filter 100 entspricht dabei im Wesentlichen dem anhand von Fig. 1 beschriebenen Filter. Gemäß diesem Ausführungsbeispiel weist der Filter 100 ein zylindrisches Filtergehäuse 104 auf. Das Konvertermaterial 108 ist in das Filtergehäuse 104 als Schüttung eines Trägermaterials 300 eingebracht. Hier ist an der Gaseintrittsöffnung 102 ein feinmaschiges erstes Gitter 302 angeordnet. Ebenso ist an der Gasaustrittsöffnung 106 ein feinmaschiges zweites Gitter 304 angeordnet. Die feinmaschigen Gitter 302, 304 verschließen den Filter gegen einen Austritt des Trägermaterials 300 aus dem Filter 100. Der Luftstrom 306 kann durch die Gitter 302, 304 im Wesentlichen ungehindert strömen.

Während des Betriebs des Filters 100 wird der Luftstrom 306 durch das Konvertermaterial 108 gefiltert. Beginnend an der Gaseintrittsöffnung 102 wird das Konvertermaterial 108 verbraucht. Es bildet sich eine Front 308 aus, die Trägermaterial 300 mit unverbrauchtem Konvertermaterial 310 von Trägermaterial 300 mit verbrauchtem Konvertermaterial 312 trennt. Im Laufe der Betriebsdauer des Filters 100 wandert die Front 308 von der Gaseintrittsöffnung 102 in Richtung der Gasaustrittsöffnung 106. Eine Geschwindigkeit der Front 308 hängt dabei von einer Menge des auszufilternden Stoffs im Luftstrom 306 ab.

Wenn die Front 308 soweit fortgeschritten ist, dass nur noch eine kritische Schütthöhe 314 von unverbrauchtem Konvertermaterial 310 verbleibt, sollte der Filter 100 durch einen unverbrauchten Filter ausgetauscht werden.

Dazu weist der Filter 100 den Sensor 110 auf. Der Sensor ist von der Gasaustrittsöffnung 106 um die kritische Schütthöhe 314 zurückversetzt. Dabei ist der Sensor 110 hier in Richtung des Luftstroms 306 gesehen vor der kritischen Schütthöhe 314 angeordnet. Das Signal des Sensors 110 zeigt damit die Front 308 an, wenn sie kurz vor der kritischen Schütthöhe 314 angekommen ist. So verbleibt ein geringer Sicherheitspuffer und der Filter 100 kann zum nächstmöglichen Termin ausgetauscht werden.

Versuche zeigen eine frontförmige Ausbreitung der gealterten Zone 312 in Gasflussrichtung über die Schütthöhe des Konverters 108. Der Sensor 110 ist im System 100 daher idealerweise so angeordnet, dass die Ankunft der Front 308 kurz vor einer funktionskritischen Restschütthöhe 314 des Konverters 108 detektiert wird, um einen rechtzeitigen Austausch zu gewährleisten.

Durch die Umwandlung des Analyten am Konvertermaterial 108 ändert sich die chemische Struktur des Konvertermaterials 108 und mit dieser die Impedanz des Materials 108. Wird eine artgleiche Beschichtung auf einen geeigneten Transducer aufgebracht, beispielsweise zwei interdigital angeordnete Kammelektroden (IDK), so kann über kontinuierliche Bestimmung der Impedanz auf die verbleibende Restaktivität zurück geschlossen werden. Eine Skizze zur Veranschaulichung dieses Prinzips ist in Fig. 4 dargestellt.

In einem Ausführungsbeispiel werden die IDK-Sensorfläche 110 sowie die ableitenden Leitungen durch Tampondruck (Polydimethylsiloxan PDMS) auf die Innenseite der Kartusche 104, in der sich später das Konvertermaterial 108 befindet, aufgebracht. Die Ableitung erfolgt durch Kontaktierung über eine Klemmung, die mittels Dichtmaterial oder Deckel hervorgerufen wird. Eine mögliche Realisierung ist eine zweigeteilte Metallisierung auf dem Verschlussstöpsel, dessen Hälften jeweils eine Seite der Elektrode ableiten. Eine Auswertung der Impedanzmessung erfolgt durch die Gerätesoftware.

Weitere Realisierungsmöglichkeiten direkt am beschichteten Trägermaterial 300 sind ebenfalls möglich. Aufgrund der durch das isolierende Trägermaterial steigenden Impedanz werden Detektoren 110 benötigt, die Impedanzen bis in den hochfrequenten Bereich von 100MHz platzsparend auflösen können.

In einem Ausführungsbeispiel erfolgt die Messung direkt am beschichteten Trägermaterial 300 über zwei gegenüberliegende Elektroden, beispielsweise Gitterelektroden. Die Elektroden sind ebenfalls kurz vor der kritischen Schütthöhe 314 an den Wänden der Kartusche 104 angeordnet, um erneut den Beginn 308 der Alterung an dieser Stelle zu detektieren.

In einem alternativen Ausführungsbeispiel erfolgt die Messung der Impedanz über die gesamte Füllhöhe. Dazu weist der Filter je eine Gitterelektrode 302, 304 zu Beginn 102 und Ende 106 der Schüttung 300 vertikal zum Gasstrom 306 auf. Durch das Fortschreiten der Alterung 308 ändert sich die Gesamtimpedanz der Konverterschüttung 300, bis ein kritischer Wert erreicht wird. So ist eine analoge Rückmeldung über die Funktionsfähigkeit des Sensors 100 möglich.

Fig. 4 zeigt eine Darstellung eines Sensors 110 gemäß einem Ausführungsbeispiel. Der Sensor 110 entspricht dabei im Wesentlichen einem Sensor, wie er in den Figuren 1 bis 3 dargestellt ist. Der Sensor 110 weist die zwei Elektroden 112, 114 auf, die durch Konvertermaterial 108 voneinander beabstandet sind. Der Sensor 110 ist als Impedanzsensor 110 ausgebildet. Die Elektroden 112, 114 sind als wechselseitig miteinander kämmende Fingerelektroden 112, 114 auf einen Sensorträger 400 aufgedruckt. Das Konvertermaterial 108 ist als durchgehende Schicht darüber auf den Sensorträger 400 aufgebracht. Dadurch ergibt sich ein mäanderförmiger Streifen Konvertermaterial 108 zwischen den Elektroden 112, 114.

Wenn das Konvertermaterial 108 verbraucht wird, ändert sich die Impedanz des Konvertermaterials 108. Die Impedanz beziehungsweise ein Wechselstromwiderstand des Konvertermaterials 108 kann durch Anlegen einer Wechselspannung 402 an die Elektroden 112, 114 gemessen werden.

Mit anderen Worten zeigt Fig. 4 ein Schema eines beschichteten Transducers 400 mit IDKs 112, 114.

Fig. 5 zeigt eine weitere Darstellung eines Sensors 100 gemäß einem Ausführungsbeispiel. Der Sensor 100 entspricht im Wesentlichen dem Sensor in Fig. 4. Der Sensor 100 weist hier zwei Beinchen 500 auf, die jeweils eine Zuleitung zu einer der Elektroden aufweisen. Der Sensorträger 400 ist vollflächig mit dem Konvertermaterial 108 beschichtet. Die Elektroden liegen hier verdeckt unter dem Konvertermaterial 108.

Mit anderen Worten zeigt Fig. 5 ein Bild eines beispielhaften Aufbaus bei Beschichtung eines keramischen Transducers 400 (Abmessungen ca. 50 x 5 x 1 mm) mit einer aufgedruckten metallischen Interdigitalstruktur nach Beschichtung mit KMnO₄ 108.

Die Beschichtung des Transducerelements 400 mit KMnO₄ 108 kann beispielsweise durch Herstellung einer Suspension von 10 mg KMnO₄ in 10 ml Aceton und ein tropfenweises Aufbringen der Suspension auf das Transducerelement 400 erfolgen. Beispielsweise können zehn Tropfen aufgetropft werden und der Sensor 100 zwischendurch trocknen gelassen werden. So kann eine Schicht 108 von ca. 10 bis 100 µm Dicke erzeugt werden.

Da sich die dünne Schicht 108 auf dem Transducer 400 analog zur Beschichtung auf dem Silicagel während der Alterung verhält, kann zur Integration in das System zum Beispiel ein IDK-Sensor 100 platzsparend in das Filtergehäuse integriert werden.

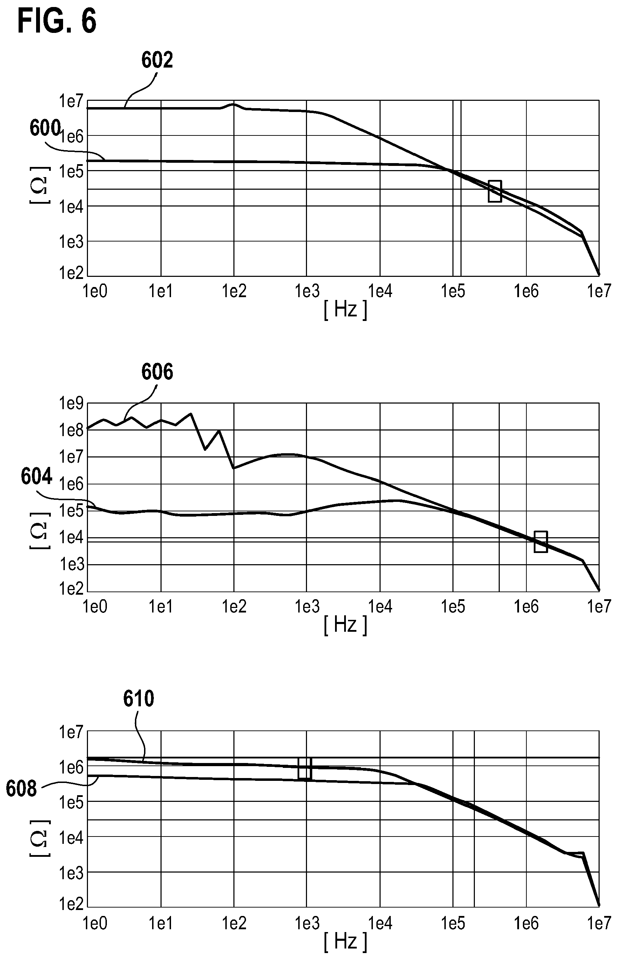

Fig. 6 zeigt Impedanzkurven 600, 602, 604, 606, 608, 610 von Impedanzsensoren gemäß einem Ausführungsbeispiel. Je zwei der Impedanzkurven 600, 602, 604, 606, 608, 610 repräsentieren einen Impedanzsensor und sind in einem gemeinsamen Diagramm dargestellt. In den Diagrammen ist auf der Abszisse eine Frequenz in Hertz [Hz] gegen eine Impedanz in Ohm [Ω] auf der Ordinate aufgetragen.

Die erste Impedanzkurve 600 repräsentiert die Impedanz eines ersten Impedanzsensors über die Frequenz, wobei das Konvertermaterial bei der ersten Impedanzkurve 600 unverbraucht ist. Bei der zweiten Impedanzkurve 602 ist das Konvertermaterial verbraucht. Deutlich ist ein Anstieg der Impedanz bei verbrauchtem Konvertermaterial erkennbar, insbesondere bei niedrigen und mittleren Frequenzen.

Die dritte Impedanzkurve 604 repräsentiert den Impedanzverlauf eines zweiten Impedanzsensors mit unverbrauchtem Konvertermaterial. Die vierte Impedanzkurve 606 repräsentiert den Impedanzverlauf des zweiten Impedanzsensors mit verbrauchtem Konvertermaterial. Auch hier ist ein deutlicher Anstieg der Impedanz bei verbrauchtem Konvertermaterial erkennbar. Dabei ist der Anstieg bei den niedrigen Frequenzen hier ausgeprägter und bei den mittleren Frequenzen weniger ausgeprägt.

Die fünfte Impedanzkurve 608 repräsentiert den Impedanzverlauf eines dritten Impedanzsensors mit unverbrauchtem Konvertermaterial. Die sechste Impedanzkurve 610 repräsentiert den Impedanzverlauf des dritten Impedanzsensors mit verbrauchtem Konvertermaterial. Die Kurvenverläufe 608, 610 entsprechen im Wesentlichen den Kurvenverläufen 600, 602, wobei hier der Anstieg der Impedanz weniger ausgeprägt, jedoch eindeutig erkennbar ist.

Die gemessene Impedanz erhöht sich bei zunehmendem Verbrauch des Materials beziehungsweise der Umwandlung von KMnO₄ in MnO₂. In Fig. 6 sind Bode-Plots zu drei unabhängig hergestellten Sensoren dargestellt. Die untere Kurve repräsentiert dabei jeweils den neuen Zustand, während die obere Kurve nach der Alterung der Probe in Gas mit NO aufgenommen worden ist.

Die Impedanzstärke im Bereich bis 1 kHz ist konstant hochohmig, durch die Alterung erhöht sich diese noch weiter und stößt an die Messbereichsgrenze des verwendeten Geräts.

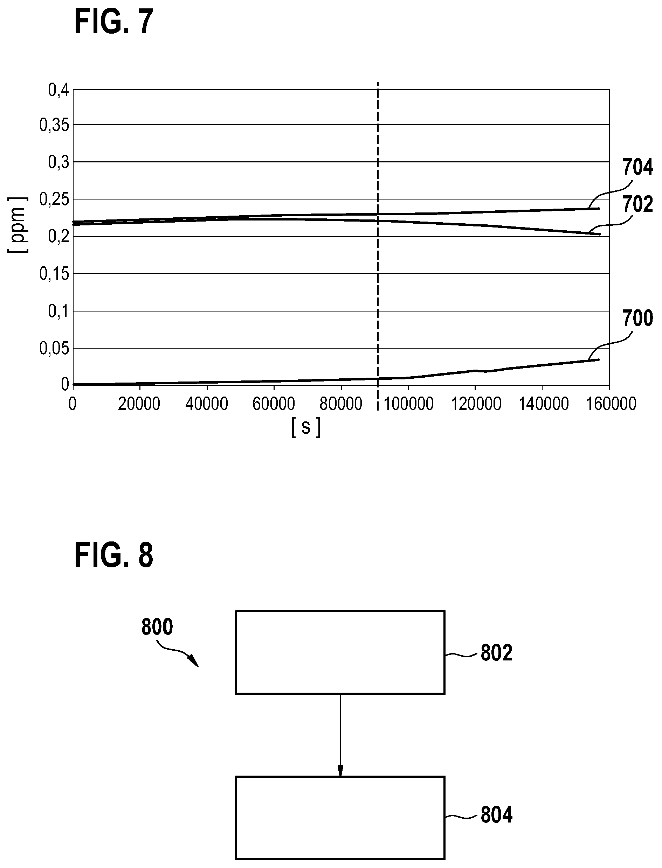

Fig. 7 zeigt Alterungskurven 700, 702, 704 eines Konvertermaterials gemäß einem Ausführungsbeispiel. Die Alterungskurven 700, 702, 704 sind in einem Diagramm dargestellt, das auf der Abszisse eine Nutzungsdauer eines Filters gemäß dem hier vorgestellten Ansatz in Sekunden [s] gegen eine Gaskonzentration in parts per million [ppm] auf der Ordinate aufgetragen hat. Dabei repräsentiert die erste Alterungskurve 700 eine Konzentration von Stickstoffmonoxid NO im unter Verwendung des Filters gefilterten Luftstrom. Die zweite Alterungskurve 702 repräsentiert eine Konzentration von Stickstoffdioxid NO₂ im gefilterten Luftstrom. Die dritte Alterungskurve 704 repräsentiert eine Konzentration von Stickoxiden NOₓ im gefilterten Luftstrom.

Die Alterungskurven 700, 702, 704 sind Chemilumineszenzdetektor (CLD)-Daten einer Messung von mit KMnO₄ beschichtetem Silicagel durch Überströmen mit NO-haltigem Gas (250 ppb NO in N₂, Gasfluss 3 l/min).

Dabei steigt die Konzentration 700 von Stickstoffmonoxid NO im gefilterten Luftstrom über die Nutzungsdauer stetig an. Die Konzentration 702 von Stickdioxid NO₂ sinkt dagegen stetig ab. Die Konzentration 704 von Stickoxiden NOₓ bleibt über die Nutzungsdauer im Wesentlichen konstant. Bei einer Nutzungsdauer von ca. 90000 Sekunden überschreitet die Konzentration 700 von Stickstoffmonoxid NO einen Grenzwert. Das deutet auf einen unvollständigen Umsatz von NO zu NO₂ durch Unterschreitung der kritischen Schütthöhe im Filter hin.

Die Alterungskurven 700, 702, 704 zeigen den typischen Alterungsmechanismus von KMnO₄ bei dauerhafter Überströmung mit einer NO-haltigen Gasmischung und verdeutlichen, an welcher Stelle der Umsatz von NO zu NO₂ durch Unterschreiten der kritischen Schütthöhe unvollständig wird.

Fig. 8 zeigt ein Ablaufdiagramm eines Verfahrens 800 zum Überwachen eines Filters beziehungsweise eines Konverters für exhaled breath analysis für ein Atemluftanalysegerät gemäß einem Ausführungsbeispiel. Das Verfahren 800 weist einen Schritt 802 des Erfassens einer Charakteristik eines Konvertermaterials des Filters auf.

In einem Ausführungsbeispiel weist das Verfahren 800 einen Schritt 804 des Bereitstellens auf. Im Schritt 804 des Bereitstellens wird eine Kapazitätsinformation des Filters und alternativ oder ergänzend eine Wartungsinformation für den Filter unter Verwendung der Charakteristik bereitgestellt.

Umfasst ein Ausführungsbeispiel eine "und/oder"-Verknüpfung zwischen einem ersten Merkmal und einem zweiten Merkmal, so ist dies so zu lesen, dass das Ausführungsbeispiel gemäß einer Ausführungsform sowohl das erste Merkmal als auch das zweite Merkmal und gemäß einer weiteren Ausführungsform entweder nur das erste Merkmal oder nur das zweite Merkmal aufweist.

## Patentansprüche

1. Filter (100) für ein Atemluftanalysegerät (200), wobei der Filter (100) die folgenden Merkmale aufweist:
ein Filtergehäuse (104), in dem ein Konvertermaterial (108) zwischen einer Gaseintrittsöffnung (102) und einer Gasaustrittsöffnung (106) angeordnet oder anordenbar ist; und
einen Sensor (110), der in dem Filtergehäuse (104) angeordnet ist und zum Erfassen einer Charakteristik zumindest eines Teils des Konvertermaterials (108) eine erste Elektrode (112) und eine zweite Elektrode (114) aufweist, zwischen denen der zumindest eine Teil des Konvertermaterials (108) angeordnet oder anordenbar ist.

2. Filter (100) gemäß Anspruch 1, bei dem der Sensor (110) mit dem zumindest einen Teil des Konvertermaterials (108) beschichtet ist.

3. Filter (100) gemäß einem der vorangegangenen Ansprüche, bei dem der Sensor (110) mit einem Abstand zu der Gasaustrittsöffnung (106) in dem Filtergehäuse (104) angeordnet ist, wobei der Abstand zumindest einer kritischen Schütthöhe (314) des Konvertermaterials (108) vor der Gasaustrittsöffnung (106) entspricht.

4. Filter (100) gemäß einem der vorangegangenen Ansprüche, bei dem die erste Elektrode (112) im Bereich der Gaseintrittsöffnung (102) angeordnet ist, und die zweite Elektrode (114) im Bereich der Gasaustrittsöffnung (106) angeordnet ist.

5. Filter (100) gemäß einem der vorangegangenen Ansprüche, bei dem die Elektroden (112, 114) als Gitterelektroden (302, 304) oder Fingerelektroden (112, 114) ausgeführt sind.

6. Filter (100) gemäß einem der vorangegangenen Ansprüche, bei dem die Elektroden (112, 114) in einer Wand des Filtergehäuses (104) ausgeformt sind.

7. Filter (100) gemäß einem der vorangegangenen Ansprüche, mit einer Impedanzerfassungseinrichtung, die dazu ausgebildet ist, eine Impedanz des zumindest einen Teil des Konvertermaterials (108) unter Verwendung des Sensors (100) zu erfassen.

8. Filter (100) gemäß einem der vorangegangenen Ansprüche, mit einer Bereitstellungseinrichtung, die ausgebildet ist, um unter Verwendung der Charakteristik des Konvertermaterials (108) ein Zustandssignal bereitzustellen, das einen Funktionszustand des Filters (100) repräsentiert.

9. Atemluftanalysegerät (200) mit einem Filter (100) gemäß einem der vorangegangenen Ansprüche.

10. Verfahren (800) zum Überwachen eines Filters (100) für ein Atemluftanalysegerät (200), wobei der Filter ein Filtergehäuse (104) aufweist, in dem ein Konvertermaterial (108) zwischen einer Gaseintrittsöffnung (102) und einer Gasaustrittsöffnung (106) angeordnet ist, und wobei das Verfahren (100) den folgenden Schritt aufweist:
Erfassen (802) einer Charakteristik zumindest eines Teils des Konvertermaterials (108) des Filters (100) unter Verwendung eines Sensors (110), der in dem Filtergehäuse (104) angeordnet ist und eine erste Elektrode (112) und eine zweite Elektrode (114) aufweist, zwischen denen der zumindest eine Teil des Konvertermaterials (108) angeordnet ist.
